# EUROPEAN PATENT APPLICATION

(11) **EP 4 772 617 A1**
(43) Date of publication of application: **08.07.2026**
(21) Application number: 24861631.0
(22) Date of filing: 28.06.2024
(51) Int. Cl.: C12N 7/01, C12N 15/54, A61K 35/761, A61K 38/45, A61K 9/00, A61P 25/28, A61P 25/00, A61P 29/00

(54) **TPK EXPRESSION VIRUS CAPABLE OF BEING ADMINISTERED THROUGH INTRAVENOUS INJECTION AND USE THEREOF IN TREATING ALZHEIMER'S DISEASE**

(30) Priority: 06.09.2023 CN 202311147204
(71) Applicant: Zhongshan Hospital, Fudan University, Shanghai 200032 (CN)
(72) Inventor: ZHONG, Chunjiu, Shanghai 200032 (CN); CENG, Yeting, Shanghai 200032 (CN); ZHAO, Xiangteng, Shanghai 200032 (CN); JIN, Boru, Shanghai 200032 (CN); CAI, Wenwen, Shanghai 200032 (CN); XIA, Yingfeng, Shanghai 200032 (CN)
(74) Representative: Sun, Yiming
(86) International application number: PCT/CN2024/102175
(87) International publication number: WO 2025/050785

(57) **Abstract**

The present invention belongs to the technical field of biomedicines, and relates to intravenous injection of an adeno-associated virus with overexpression of thiamine pyrophosphokinase-1 (TPK). The adeno-associated virus with overexpression of thiamine pyrophosphokinase-1 is a recombinant adeno-associated virus, and the genome of the adeno-associated virus with overexpression of thiamine pyrophosphokinase-1 comprises a neuron-specific promoter, a nucleotide sequence encoding a TPK gene, and a WPRE element. The present invention also provides a method for constructing AAV-TPK and its application in the preparation of a medicament for preventing or treating Alzheimer's disease. In the present invention, the serotype of AAV-TPK is AAV.CAP-B10, which can specifically and efficiently cross the blood-brain barrier and widely act on all brain regions. By means of peripheral administration, specifically brain targeting and high activity of exogenous TPK overexpressing, the present invention ensures convenience of administration while avoiding potential side effects caused by peripheral TPK overexpression. In clinical applications, the AAV-TPK of the present invention can be administered by direct peripheral intravenous injection to across the blood-brain barrier effectively, thereby treating or preventing Alzheimer's disease.

## Description

### TECHNICAL FIELD

The present invention relates to intravenous injection of an adeno-associated virus with overexpression of thiamine pyrophosphokinase-1 and its application in treating Alzheimer's disease, and belongs to the technical field of biomedicines.

### BACKGROUND

Alzheimer's disease (AD) refers to a serious neurodegeneration, which commonly occurs in elderly people and is mainly clinically manifested as impaired memory, reduced language ability and spatial orientation ability, mental disorder, etc. Pathologically, it is usually accompanied by a large number of neuronal deaths, which further lead to brain atrophy. At present, there are the following hypotheses about the pathogenesis of AD: β-amyloid (Aβ) cascade hypothesis, Tau hypothesis, neuroinflammatory hypothesis, oxidative stress hypothesis, and so forth. Although scientists have carried out extensive research based on relevant hypotheses, the clinical efficacies of the current therapeutic methods and drugs against AD still are limited.

Brain accounts for about 2% of the body weight, but the energy it consumes accounts for about 20% of the total energy consumed by the human body. Glucose is the dominant substrate for energy metabolism in the brain. Therefore, glucose metabolism is very important for the brain to function normally. In previous studies, it was found that abnormal glucose metabolism in brains of AD patients occurs decades before its clinical symptoms and it is closely related to the degree of cognitive decline and the progression of the disease. Therefore, it is expected to regulate the level of glucose metabolism in brain cells to delay or end the progression of AD.

Thiamine, also known as vitamin B1, is transformed into biologically active thiamine diphosphate (TDP) by thiamine phosphokinase-1 (TPK) in the body. TDP is an essential coenzyme for transketolase, pyruvate dehydrogenase, and α-ketoglutarate dehydrogenase, which these three enzymes are important in glucose metabolism. The previous studies have shown that in the brain and blood samples of AD patients, the activities of transketolase, pyruvate dehydrogenase, and α-ketoglutarate dehydrogenase are significantly decreased, Further, the levels of blood and brain TDP in AD patients are significantly lower as compared with those in control subjects with normal function, and contribute to the decline of brain glucose metabolism of the AD patients. Furthermore, the levels of TPK mRNA in the brain samples of AD patients are significantly lower than those in control subjects with normal brain function, Frontotemporal degeneration, Parkinson's disease, Huntingdon's disease, and amyotrophic lateral sclerosis. In the brain of the AD patients, the level of TPK mRNA is negatively correlated with Braak scores of pathological grading, but in the brain tissues of individuals with normal cognitive function, the level of TPK mRNA is positively correlated with the Braak scores of pathological grading. Also, the levels of TPK protein in brains of AD patients are significantly decreased as compared with that in control subjects. The results show that TPK expression may be a protective factor that delays the occurrence and progression of AD. Importantly, conditional knockout of the TPK gene in excitatory neurons of adult mice significantly replicates all important pathological features of human AD, such as abnormal brain glucose metabolism and cognitive dysfunction, brain atrophy caused by progressive loss of synapses and neurons, Aβ deposition, Tau hyperphosphorylation and neurofibrillary tangle formation, activation of microglia and astrocytes and neuroinflammation, impairment of microvascular formation, and dysregulation of peripheral glucose metabolism. (With reference to, for example, Sang et al., Thiamine pyrophosphokinase deficiency induces Alzheimer's pathology, Biorxiv, 2020).

Adeno-associated virus (AAV) is currently the simplest non-enveloped single-stranded DNA virus. Recombinant AAV, due to its high safety, low immunogenicity, broad host range, and stable expression, is widely used as a vector for gene therapy. Currently, there are more than 250 clinical projects of AAV gene therapy carried out worldwide, and U.S. FDA has also approved two AAV gene therapies, namely Luxturna that is administered via subretinal injection, and Zolgensma that is administered via intravenous injection, and these two products are used for treating Leber's congenital amaurosis and pediatric spinal muscular atrophy, respectively.

At present, it is urgently needed to develop novel methods for treating AD. In recent years, gene therapy using recombinant adeno-associated virus (rAAV) has emerged, with targets mainly focusing on Aβ metabolism-related proteins: APP, Bace1, γ-secretase, Tau protein, ApoE, etc. There have been no reports on gene therapy strategies that regulate energy metabolism. On the other hand, current gene therapy methods for AD mostly include injections into the brain or ventricles, or subarachnoid space, which have disadvantages such as limited infected brain regions, complex operations, and brain injection injuries.

### SUMMARY

The present invention aims at solving the technical problems that existing gene therapy methods have disadvantages of limited infected brain regions, and brain injection injuries, and provides a recombinant adeno-associated virus (rAAV) which can specifically target in brain and overexpress TPK (thiamine pyrophosphokinase-1) through peripheral administration, which is convenient and can avoid potential side effects of peripheral overexpression of TPK, and its application thereof in treating Alzheimer's disease (AD). The rAAV in a genome includes an expression cassette, and the expression cassette includes polynucleotide which is operably connected with a promoter and is used for encoding human *TPK* gene.

In order to achieve above aim, the present invention provides intravenous injection of an adeno-associated virus with overexpression of thiamine pyrophosphokinase-1, the adeno-associated virus with overexpression of thiamine pyrophosphokinase-1 is a recombinant AAV, and its genome includes a neuron-specific promoter, a nucleotide sequence encoding a TPK gene, and a WPRE element.

Further, the serotype of AAV is AAV.CAP-B10, the neuron-specific promoter has a gene sequence shown as SEQ ID NO: 2, the nucleotide sequence encoding the TPK gene is shown as SEQ ID NO: 3, and the WPRE element has a sequence shown as SEQ ID NO: 4.

Further, the sequence of the recombinant AAV is shown as SEQ ID NO: 1.

The present invention provides a method for constructing an adeno-associated virus with overexpression of thiamine pyrophosphokinase-1, and the method includes the following steps:
step 1: obtaining a TPK target gene by a PCR amplification or restriction endonuclease digestion method, and performing homologous recombination using Exnase recombinase, then assembling with an adeno-associated virus vector by a T4 DNA linkage method;
step 2: transforming the virus vector assembled in step 1 into E.coli Stbl3 competent cells;
step 3: screening a transformant by colony PCR, sequencing positive clones, and determining the positive clones by sequence alignment; and
step 4: the screened and identified virus vector together with pHelper plasmid and pAAV-R plasmid co-transfecting with AAV-293 cells, and then performing packaging and purifying of the AAV.

Further, primers for the PCR amplification in step 1 or colony PCR in step 3 are a 4423-BamHI-F primer and a 4423-SalI-R primer, the sequence of the 4423-BamHI-F primer is shown as SEQ ID NO: 5, and the sequence of the 4423-SalI-R primer is shown as SEQ ID NO: 6.

Further, a forward sequencing primer and a reverse sequencing primer for transformant sequencing in step 3 are a hSyn-C-F primer and a pEGFP-N-3' primer respectively, the sequence of the hSyn-C-F primer is shown as SEQ ID NO: 7, and the sequence of the pEGFP-N-3' primer is shown as SEQ ID NO: 8.

The present invention provides a kit for preparing the adeno-associated virus with overexpression of thiamine pyrophosphokinase-1 (TPK); the kit includes the 4423-BamHI-F primer and 4423-SalI-R primer which are used for PCR amplification, the sequence of the 4423-BamHI-F primer is shown as SEQ ID NO: 5, and the sequence of the 4423-SalI-R primer is shown as SEQ ID NO: 6.

Further, the kit also includes the forward sequencing primer and the reverse sequencing primer for detecting the positive clones, the forward sequencing primer is the hSyn-C-F primer and has the sequence shown as SEQ ID NO: 7, and the reverse sequencing primer is the pEGFP-N-3' primer and has the sequence shown as SEQ ID NO: 8.

The present invention provides an application of the adeno-associated virus with overexpression of thiamine pyrophosphokinase-1 (TPK) in preparation of a medicament for preventing or treating AD.

Further, the present invention provides an application of the adeno-associated virus with overexpression of thiamine pyrophosphokinase-1 (TPK) in preparation of a medicament for preventing or treating AD-like neurodegeneration, brain atrophy, Aβ and tau pathological changes, neuroinflammation, and neurovascular disorders, or slowing down progression of AD.

Further, the prepared medicament is administrated by peripheral intravenous injection, which can target neurons across the blood-brain barrier.

Compared with the prior art, the present invention has the following beneficial effects:
1. AD refers to across multiple brain regions lesions, and the serotype rAAV in the present invention is AAV.CAP-B10.hsyn-TPK, which can specifically and efficiently cross the blood-brain barrier and widely act on all brain regions. In clinical application, the rAAV in the present application can be directly administrated by peripheral intravenous injection for treating or preventing AD; the administration method is simple and convenient, has no obvious injury or other side effects, and has the advantages of avoiding potential side effects by peripheral overexpression of TPK, etc.
2. The defects such as complex operation and obvious injuries in other serotype AAV injection operations, for example direct injection into ventricles, subarachnoid space or brain parenchyma for therapy are effectively avoided by using the method in the present application.
3. The AAV.CAP-B10.hsyn-TPK in the present invention is different from other gene therapy in that TPK in neuron is compensated to enhance brain energy metabolism to treat AD.
4. The AAV.CAP-B10.hsyn-TPK in the present invention only exogenously mediates compensatory expression of TPK, does not involve endogenous TPK, and even does not involve editing of other genes, off-target risk of gene editing can be greatly reduced.
5. The AAV.CAP-B10.hsyn-TPK in the present invention introduces highly active exogenous TPK, achieving more beneficial treatment effect.

### BRIEF DESCRIPTION OF THE DRAWINGS

FIG. 1 is a plasmid profile of a rAAV genome before insertion of a TPK target gene in an Example 1.
FIG. 2 is a plasmid profile of the rAAV genome after insertion of a TPK target gene in an Example 1.
FIG. 3 shows a representative image of DNA gel electrophoresis detection on PCR amplification products in an Example 2.
FIG. 4 shows a representative image of DNA gel electrophoresis detection on colony PCR amplification products in an Example 2.
FIG. 5 shows a comparison image of high-throughput sequencing on a transformant in an Example 2.
FIG. 6 shows a result of mouse alternation ratio test in a Y-maze test in an Example 3.
FIG. 7 shows a result of mouse running end speed test in a rotarod test in an Example 3.
FIG. 8 shows a result of mouse nesting score test after 24 hours in a nesting test in an Example 3.
FIG. 9 shows a result of mouse nesting score test after 48 hours in a nesting test in an Example 3.
FIG. 10 shows a result of weights of mouse half brain, cortex, and hippocampus in an Example 4.
FIG. 11 shows a result of superficial area of left half brain of mice in an Example 4.
FIG. 12 shows a representative image and a quantitative result of TPK protein in a mouse cortical sample subjected to Western blotting detection in an Example 5.
FIG. 13 shows a representative image and a quantitative result of TPK protein in a mouse cortical sample subjected to immunofluorescence staining detection in an Example 5.
FIG. 14 is a representative confocal image of mouse brain sections after brain infection with an AAV.CAP-B10 virus in an Example 6.
FIG. 15 is representative confocal images of mouse liver, heart, kidney, and testis tissue sections after infection with an AAV.CAP-B10 virus in an Example 6.
FIG. 16 shows a representative image of Western blotting detection on APP, BACE1, Tau, and p-Tau (S396) in an Example 7.
FIG. 17 shows quantitative results of Western blotting detection on APP, BACE1, Tau, and p-Tau (S396) in an Example 7.
FIG. 18 shows a representative image and a quantitative result of immunofluorescence staining with NeuN antibody in an Example 7.
FIG. 19 shows a representative image and a quantitative result of immunofluorescence staining with 4G8 and Iba1 antibody in an Example 7.
FIG. 20 shows a representative image and a quantitative result of immunofluorescence staining with GFAP antibody in an Example 7.

### DETAILED DESCRIPTION

Through the following embodiments, those skilled in the art will better understand the present invention. It is to be understood that the following embodiments are intended to exemplify the embodiments of the present invention, rather than to limit its scope. Unless otherwise specified, the methods employed in the present invention are conventional methods in the art, and the experimental materials used are commercially available.

### Example 1: Preparation of AAV.CAP-B10.hsyn-TPK rAAV.

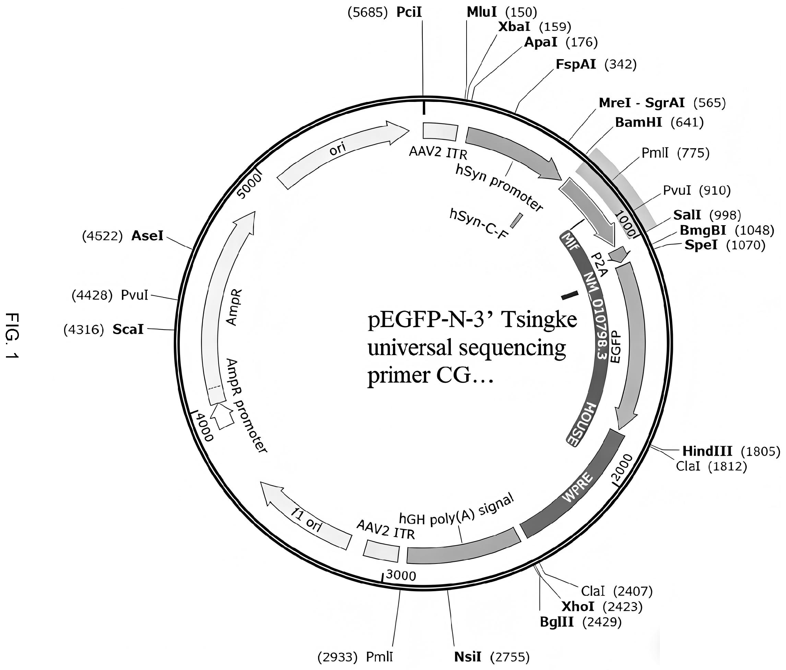

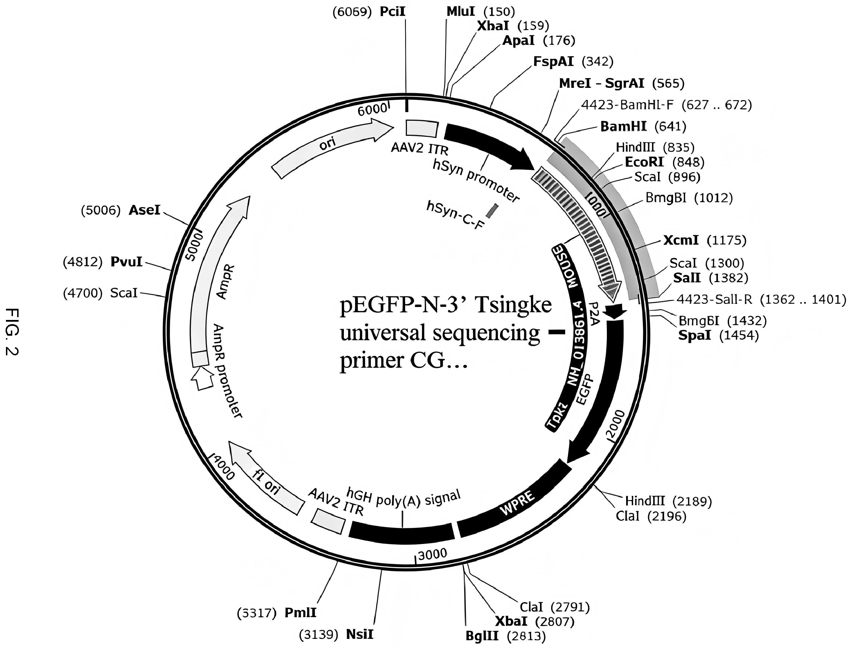

The CRO Company, Wuhan BrainVTA Neuroscience Technology Co., Ltd., is commissioned to prepare laboratory-scale AAV (serotype: AAV-CAP-B10) which can encode TPK, hereinafter referred to as AAV-TPK, and its genome contains: i) a neuron-specific promoter (SEQ ID NO: 2); ii) a nucleotide sequence (SEQ ID NO: 3) encoding mouse TPK and a WPRE element (SEQ ID NO: 4). FIG. 1 and FIG. 2 are plasmid profiles (including restriction sites) of the r AAV genome before and after insertion of a TPK target gene, respectively.

### I. Test methods

A method for constructing a rAAV vector is as follows:
step 1: obtaining a TPK target gene by a PCR amplification and restriction endonuclease digestion method, and performing homologous recombination using Exnase recombinase, then assembling with an adeno-associated virus vector by a T4 DNA linkage enzyme;
step 2: transforming the virus vector assembled in step 1 into E.coli Stbl3 competent cells; and
step 3: screening a transformant by colony PCR, sequencing positive clones, and conforming the positive clones by sequence alignment.

The information of agents, instruments, and vector used in the construction method are as follows.

### 1. Main agents

The main agents used in this example are listed in Table 1.

**Table 1**

| Agent name | Manufacturer | Item No. |
|---|---|---|
| *Trans* 2K^{®} Plus II DNA Marker | Transgen | BM121-01 |
| Agarose | Seven Biotech | SM111-01 |
| Basic Seamless Cloning Kit | Transgen | CU201-03 |
| 2×Mix (Dye Plus) | Vazyme | P525-AA |
| Primer | Tsingke Biotech | |
| Restriction endonuclease | Thermo | |
| Plasmid extraction kit | iGeneBio | P001-02 |
| Agarose gel DNA recovery kit | iGeneBio | G001-01 |

### 2. Main instruments

The main instruments used in this example are listed in Table 2.

**Table 2**

| Name of instrument | Manufacturer | Item No. |
|---|---|---|
| PCR instrument | Jingle Scientific Instrument | K960 |
| DNA electrophoresis apparatus | Liuyi Biotechnology | DYY-7C type |
| Gel imager | TIANNENG | Tanon 2500 |
| Bacterial shaker | ZHICHENG Inc. | ZYW-2102C |
| Bacterial incubator | ZHICHENG Inc. | ZXDP-B2080 |
| High-speed centrifuge | Hunan Xiangyi | H1850R |

### 3. Plasmid information

The vector plasmid information in this example is listed in Table 3.

**Table 3**

| | | | | |
|---|---|---|---|---|
| Product No. | TSG-01 | Name of gene: | Tpk1 | |
| GenBank ID: | NM_013861.4 | CDS size: | 729bp | |
| Species: | MOUSE | Upstream and downstream cloning restriction sites | BamHI | SalI |
| Prokaryotic resistance | Amp | | | |
| Name of vector: | rAAV-hSyn-Tpk1-P2A-EGFP-WPRE-hGH polyA | | | |
| Way of expression | 2A links two genes for non-fusion expression | | | |
| Virus sequence information | SEQ ID NO: 1 | | | |
| Other Instructions | 2A peptide (2A self-cleaving peptides): | | | |
| | 1. Definition: It is a type of peptide fragment having 18-22 amino acid residues, and it is capable of inducing recombinant proteins containing 2A peptide within cells to perform self-cleaving. Such peptides have a sequence motif section, which often causes ribosome to fail to link at the junction of glycine (G) and proline (P) at the last, resulting in a "cleaving" effect. | | | |
| | 2. Among the four common 2A peptides (P2A, T2A, E2A, and F2A), P2A generally has higher cleaving efficiency. Adding a GSG (Gly-Ser-Gly, glycine, serine, and glycine) sequence to an N-terminus of 2A peptide sequence can enhance the efficiency of 2A peptide-induced cleaving. | | | |
| | 3. P2A (Porcine teschovirus) (GSG) sequence is ATNFSLLKQAGDVEENPGP (SEQ ID NO: 9), with the cleaving site between Gly-//-Pro. | | | |

### 4. Primers for PCR amplification

The primers sequences used for PCR amplification in step 1 of the construction method in this example are listed in Table 4. The primers comprise exchangeable paired bases, restriction sites, as well as a part of the 5' end sequence of the target gene for obtaining the target gene by PCR.

**Table 4**

| ID | seq |
|---|---|
| 4423-BamHI-F | agcgcagtcgagaaggatccgccaccatggagcatgcctttacccc (SEQ ID NO: 5) |
| 4423-SalI-R | gtagctccgcttccgtcgacgctcttgatggccatggtcc (SEQ ID NO: 6) |

### 5. PCR amplification reaction system

After DNA extraction, the following recommended components are added into an RNase-free PCR tube on ice, mixed thoroughly, and centrifuged briefly:

| Components | Addition amount |
|---|---|
| DNA polymer | Complement to 20 µL |
| 4423-BamHI-F | 1 µL |
| 4423-BamHI-R | 1 µL |

After mixing, PCR cycle program is set and the samples are loaded into the thermal cycler. The PCR cycling steps are as follows:

| Steps | Temperature | Time | Number of cycles |
|---|---|---|---|
| Heating | 94°C | 3 min | 1 |
| Denaturation | 94°C | 30 s | 35 times |
| Annealing | 63°C | 30 s | |
| Elongation | 72°C | 50 s | |
| Final elongation | 72°C | 5min | 1 |
| Storage | 10°C | Hold | ∞ |

### II. Test results

### 1. PCR results of target gene

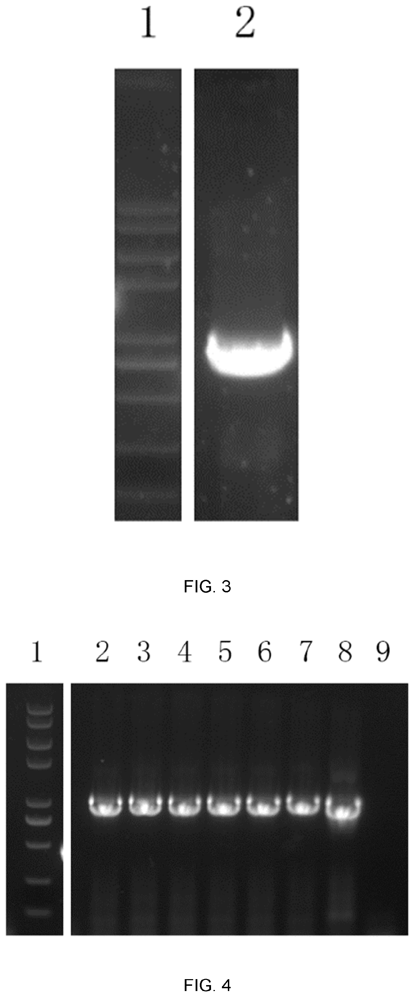

In step 1 of the above construction method, the TPK target gene is obtained by PCR amplification, and the PCR amplification product is detected by DNA gel electrophoresis, and the results are shown in FIG. 3.

In FIG. 3, Lane 1: Trans2K^{®} Plus II DNA Marker (bands from top to bottom are: 8 kb, 5 kb, 3 kb, 2 kb, 1 kb, 750 bp, 500 bp, 250 bp, and 100 bp) recombinant plasmid construction; Lane 2: amplified bands of the target fragment.

The results in FIG. 3 show that the DNA molecular weight of the PCR amplification product is 775 bp, indicating that the target gene is successfully amplified.

After successful PCR amplification of the target gene, the linearized vector and the target gene fragment are mixed proportionally, and the homologous recombination linkage between the target gene fragment and the vector is performed using Exnase recombinase.

### 2. PCR results of transformants

In step 3 of the above construction method, the transformants are screened by the colony PCR; and the PCR identification primer sequences are listed in Table 5. The colony PCR amplification products are detected by DNA gel electrophoresis, with the results shown in FIG. 4.

**Table 5**

| ID | seq |
|---|---|
| 4423-BamHI-F | agcgcagtcgagaaggatccgccaccatggagcatgcctttacccc (SEQ ID NO: 5) |
| 4423-SalI-R | gtagctccgcttccgtcgacgctcttgatggccatggtcc (SEQ ID NO: 6) |

In FIG. 4, Lane 1: Marker from top to bottom is 8 kb, 5 kb, 3 kb, 2 kb, 1 Kb, 750 bp, 500 bp, 250 bp, 100 bp; Lanes 2-9: Transformants 1-8.

The results in FIG. 4 show that the DNA molecular weight of the colony PCR amplification products is 775 bp, indicating that the transformants contain the target gene fragment and are positive clones.

### 3. Sequencing results of transformants

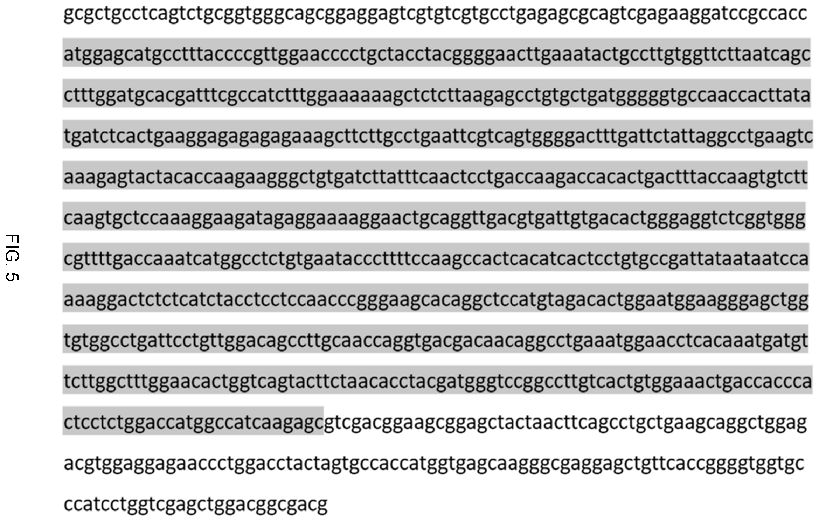

High-throughput sequencing is performed to validate the sequencing of the above positive clones transformants; the sequences of primers are listed in Table 6, and the sequencing of positive clones transformants alignment results are shown in FIG. 5, in which, the color-marked parts indicate the target gene sequences.

**Table 6**

| | |
|---|---|
| Forward sequencing primer | hSyn-C-F: gcgctgcctcagtctgc (SEQ ID NO: 7) |
| Reverse sequencing primer | pEGFP-N-3': cgtcgccgtccagctcgaccag (SEQ ID NO: 8) |

The sequence alignment results are shown in FIG. 5 and indicate the presence of the target gene fragment in the positive clones transformants screened by colony PCR, confirming them as positive clones transformants.

### Example 2: Packaging of rAAV

### 1. Preparation of plasmids

The viral vectors (viruses carrying exogenous genes) screened and identified in the Example 1, the pHelper plasmid (carrying adenovirus-derived genes), and the pAAV-RC plasmid (carrying AAV replication and capsid genes) were extracted on a large scale.

### 2. AAV-293 cell culture

### (1) Recovery of AAV-293 cells

A medium was preheated, and a cell cryopreservation tube was quickly transferred from a liquid nitrogen container into a water bath filled with water at 37°C, and the cell cryopreservation tube was shaken from time to time for unfreezing as soon as possible; then the cell cryopreservation tube was wiped and disinfected with 70% alcohol and transferred to an ultra-clean workbench; a cell suspension was extracted and added into 3 ml of medium, then 5 ml of medium was added to dilute and was gently blown to be dispersed; the cells were collected by centrifuging at 1,000 rpm for 3 min; and the cells were resuspended with a proper amount of media for plating.

### (2) AAV-293 cell passage (10cm vessel taken as an example)

The used culture solution was removed, 10 ml of sterilized PBS was added and gently shaken, the cell growth surfaces were washed, and then the PBS was removed; 1 ml of pancreatic enzyme digestion solution was added to digest for 1-2 min until the cells were rounded and begun to fall off; 2 ml of a complete medium was added to end the digestion, and the cells at the bottom of the vessel were blown and beat into single cells; and the cells were subjected to centrifuging, resuspending, counting and plating as needed, or the cells were mixed thoroughly and then directly distributed into a new flask to be continually cultured.

### (3) Cryopreservation of AAV-293 cells

The cells that grew vigorously after being cultured for 2-3 days were collected; the cells were digested and then blown and beat into the single cells according to the passaging step; the cells were collected by centrifuging at 1,000 rpm for 3 min; a proper amount of PBS was added to resuspend the cells, and then the cells were counted; the cells were collected by centrifuging at 1,000 rpm for 3 min; the cells were resuspended with a cryopreservation medium until reaching 2x10⁶-5x10⁶ cells/ml, and then the cell suspension was subpackaged into 2ml cryopreservation tubes, 1 ml per tube; and the small tubes were put into a program cassette, and the cassette was frozen at temperature of -80°C overnight; and the small tubes with the cryopreserved cells were transferred into liquid nitrogen for long-term storage the next day.

### 3. Cell transfection

### (1) Plating of AAV-293 cells

The AAV-293 cells were plated in a 10cm vessel and transfected after 48 h, and the confluency reached 70-80% during transfection.

The AAV-293 cells were transfected; a cell culture plate was taken out 1 h before transfection, the original cell medium was removed, and 10 ml of an Opti-MEM medium was added.

A complex of a transfection agent and the plasmids was prepared as follows: 32 µg of virus vector plasmids to be transfected (pHelper: pAAV-RC: virus vector = 1:1:1) were dissolved in the Opti-MEM medium with a total volume of 500 µl, and were lightly mixed thoroughly , and then the mixture was stood for 5 min; the transfection agent was dissolved in the Opti-MEM medium with a total volume of 500 µl, and was lightly and mixed thoroughly, and then the mixture was stood for 5 min; and the transfection agent diluent was dropwisely added to the plasmid diluent, accompanied by light and thorough mixing, and then the mixture was stood at room temperature for 20 min to fully combine the DNA and the transfection agent to form a stable transfection complex.

After the complex of the transfection agent and the plasmids was prepared, the cell vessel was taken out, and the prepared DNA-transfection agent complex was added to the cell culture plate.

### (2) Solution change after transfection

The medium was extracted 6 h later, and PBS was added to wash once, and then 10 ml of a fresh complete medium was added for culturing.

### 4. Collection and purification of virus

(1) After 60 h of transfection, the cells were scraped off using a cell scraper and were then collected into a centrifuge tube; and the cells were centrifuged at 1,500 rpm under 4°C for 5 min. Cell precipitates were resuspended in 9 ml of lysis buffer, and a culture solution was collected into a 500ml flask; the cell lysis buffer was placed in liquid nitrogen and frozen for 3 min, and then the cell lysis buffer was transferred into a water bath at 37°C to be repeatedly freeze-thawed 3-4 times; then 250 U of benzonase was added and mixed thoroughly, and then the mixture was cultured at 37°C for 1 h;
(2) NaCl was added until the final concentration reached 150 mM; after thorough mixing, the mixture was cultured at 37°C for 30 min; 2,500g of the mixture was centrifuged at room temperature for 10 min, then the supernatant was collected, and the precipitate was removed; the centrifuged supernatant was placed into the supernatant that was centrifugally collected in step (1), PEG8000 and NaCl were added until the final concentrations reached 8% and 0.5M, respectively, and the solution was stood at 4°C overnight; the solution was centrifuged at 12,000 rpm under 4°C for 1.5 h, then the supernatant was removed, and the precipitate was resuspended with 10 ml of PBS; the resuspended virus stock solution was added to an ultracentrifuge tube that was filled with iodixanol (iodixanol concentrations of 15%, 25%, 40%, and 60% respectively), and the solution was centrifuged at 63,000 rpm under 18°C for 2 h;
(3) 40% of layered sample was extracted using a 10ml injector, and the sample was put into a dialysis bag for dialyzing at 4°C; the dialysate was changed after 12-16 h, with 2 L of buffer solution used each time; and the dialyzed sample was collected and concentrated using an ultrafiltration tube.

### 5. Titer determination

Currently, the titer of AAV is determined by quantitative PCR measuring the genome copies of the AAV vector in the genome in order to determine the number of AAV viral particles. The specific steps are as follows:
preparation of a standard substance: a plasmid standard substance is utilized, the concentration of the standard substance is calculated, it is diluted to be 1E+8 viruses/µL, and gradient dilution is carried out until reaching 1E+3 viruses/µL, with a total of 6 gradients; and
preparation of samples: alkaline lysis is carried out, 5 µl of concentrated virus sample is collected and added with NAOH until reaching the final concentration of 1 M; after processing in a water bath at 55°C for 30 min, HCl is added to neutralize, and pure water or 1XPBS is added to dilute 10 times.

18 µl of reaction solution is added to each reaction well, and then 2 µl of template is added; and the reaction solution is loaded into the machine, the annealing temperature is set to be 60°C, the operation is carried out according to standard to obtain a Ct value, and the genome copies in the AAV sample are calculated.

### Example 3. Tail intravenous injection of AAV-TPK on improvement of cognitive behavior of cKO mice

The purpose of this example is to verify whether the gene therapy can significantly improve the expression level of TPK protein in the brains of CaMKII-CreERT2/+;Tpkfl/fl mice and alleviate cognitive deficits in transgenic mice by tail intravenous injection of AAV.

3-month-old CaMKII-CreERT2/+;Tpkfl/fl mice (cKO) and littermate control mice (WT) were induced with tamoxifen (30 mg/kg) and were randomly divided into two groups (WT-Ctrl, n=9; WT-TPKovp, n=12; cko-Ctrl, n=9; cKO-TPKovp, n=11).

After 4 weeks of tamoxifen induction, the mice in each group were administrated by tail intravenous injection with suspensions of AAV-TPK at a titer of 2.5E+12vg/mL (prepared with PBS) (WT-TPKovp group and cKO-TPKovp) and control rAAV (identical to TPK-AAV except without the TPK encoding sequence), 200 µL per mouse. Specifically, the mice were fixed with a tail intravenous injection restrainer, the tails were disinfected with alcohol swabs, and 200 µL of the viral suspension was injected into the tail vein using a 1 mL syringe; and after injection, a dry cotton ball was pressed on the injection site for 30 sec to prevent leakage of the virus. After being injected with the virus, the mice were continuously fed for 4 weeks, then, all mice were subjected to behavioral test, biochemical measurement, and pathological examination. In this example, the cognitive behavioral assessment includes Y-maze test, rotarod test, and nesting test; and the specific experimental methods are as follows.

### I. Test methods

### 1. Y-maze test

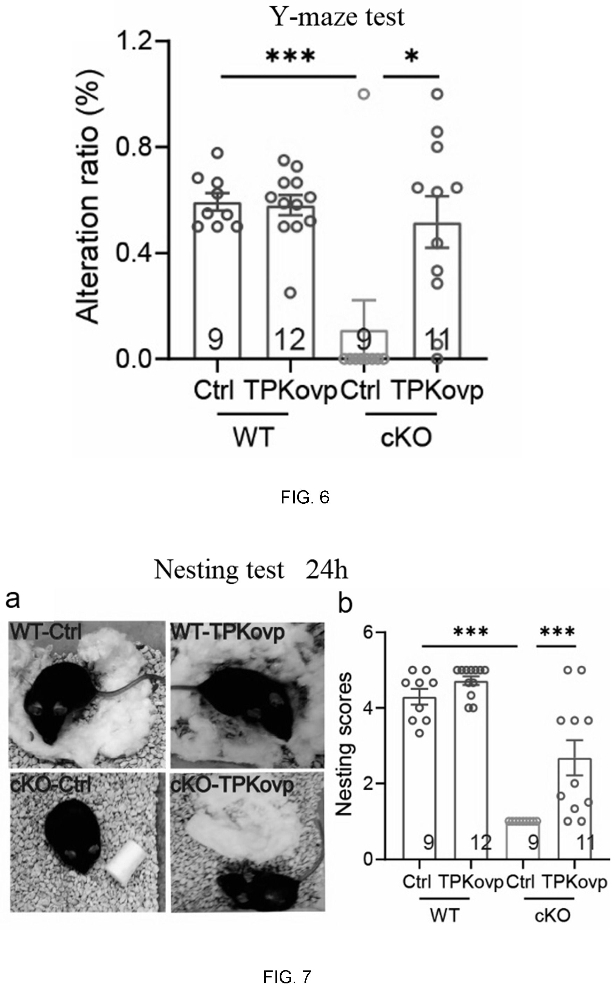

All mice were subjected to the Y-maze test by conventional methods. The results are shown in FIG. 6.

### 2. Rotarod test

The rotarod had a diameter of 3 cm and a lane width of about 8 cm. On the day of testing, the mice were allowed to acclimate to a test room for 30 min before the test. After 30 min of acclimation, the mice were placed on a runway, and once the mice stood stably, the rotarod was rotated at a constant speed of 4 rpm, with the acclimation time of 120 sec. After a batch of mice acclimated, each runway was disinfected with 75% alcohol and wiped dry before acclimation of the next batch. After all the mice acclimated, the final test was carried out with at least a half-hour interval. During the test process, the runway was set to an initial speed of 4 rpm, with an acceleration time of 5 s, lasting for 10 s; then the rotarod was accelerated from 4 rpm to 35 rpm within 240 s, and maintained at 35 rpm for 60 s, and the speed at which the mice fell from the rotarod was recorded. The results are shown in FIG. 7.

### 3. Nesting test

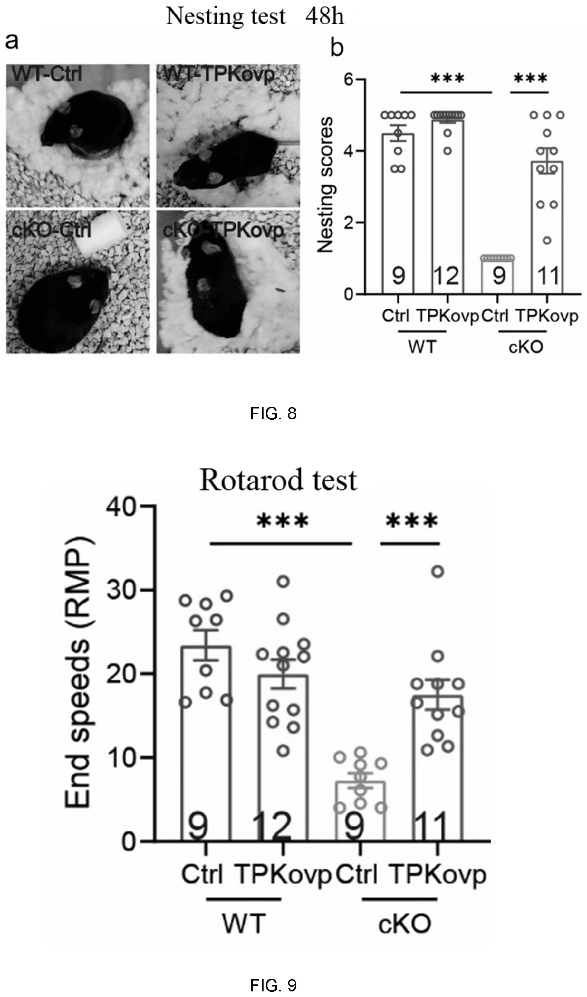

The nesting behavioral test is commonly used for assessing the daily activities of mice with cognitive impairments, and in this test, it can evaluate the fine movement flexibility, cognition, and emotional state while applying minimal stress to the mice. The materials for nesting are cylindrical cotton fibers with a diameter of 3 cm and a length of 3 cm. Before the test, the mice were fed in cages individually, and all materials for nesting were removed from the cages. The materials for nesting were placed in the cages, adequate food and water were provided, and nesting scores were assessed 12 h later, with the criteria listed in Table 7. FIG. 8 and FIG. 9 show the nesting scores of the mice 24 h and 48 h after the start of the test, respectively.

**Table 7**

| Score | Description |
|---|---|
| 1 | The material remains more than 90% intact. |
| 2 | Some of the materials are torn apart, with more than 50% remaining intact. |
| 3 | Most of the materials are torn apart, with less than 50% remaining intact, but the intact materials are scattered and not built into a nest. |
| 4 | Most of the materials are torn apart, with only 10% remaining intact and built into a recognizable, but not collapsed, horizontal nest. |
| 5 | The materials were completely torn into a crater-shaped depression nest. |

### II. Test results

The results in FIG. 6 show that compared with the littermate control mice, the cKO mice in the Y-maze test have a significant decrease in alternation ratio, while the alternation ratio of the cKO mice subjected to TPK overexpression treatment is significantly improved.

The results in FIG. 7 show that compared with the littermate control mice, the cKO mice in the rotarod test have a significant decrease in end speed of running, while the end speed of running of the cKO mice subjected to TPK overexpression treatment is significantly improved.

The results in FIG. 8 and FIG. 9 show that compared with the littermate control mice, the cKO mice in the nesting test have a significant decrease in nesting scores 24 h and 48 h after the start of the nesting test, while the nesting scores of the cKO mice subjected to TPK overexpression treatment are significantly improved.

Conclusion: Tail intravenous injection of AAV.CAP-B10.hsyn-TPK can improve the cognitive function of the cKO mice.

### Example 4. Tail intravenous injection of AAV.CAP-B10.hsyn-TPK on alleviation of brain atrophy of cKO mice

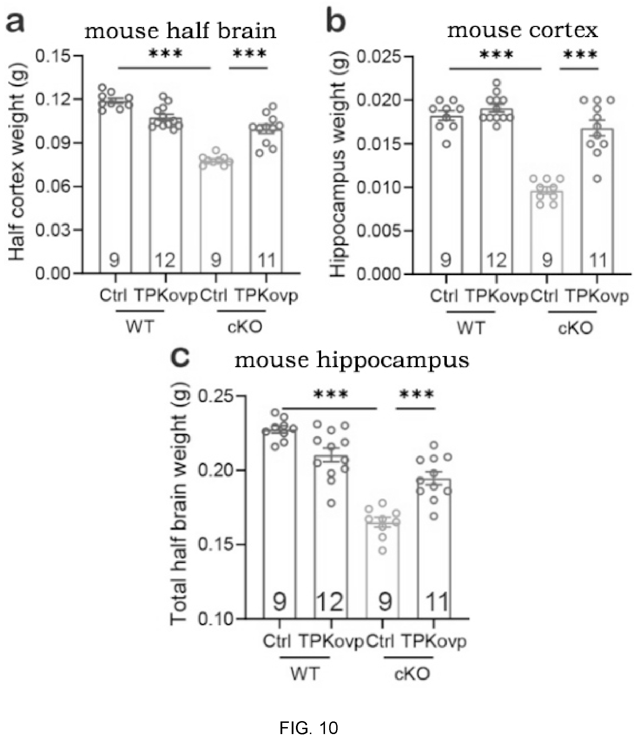

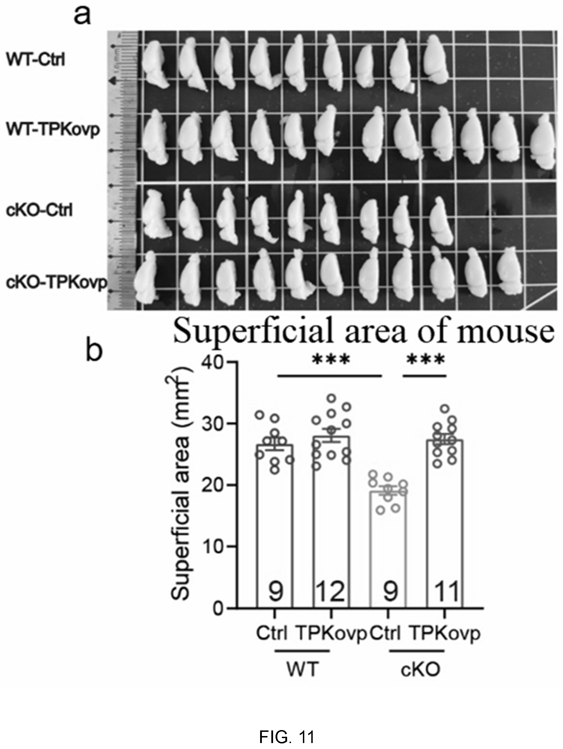

After the mouse behavioral test, the mice were euthanized, followed by cardiac perfusion using pre-cooled PBS; and after the blood was completely flushed out, the left half brain tissue of the mice was collected (excluding the cerebellum, brainstem, and olfactory bulb, with the left and right brains separated). The weights of the half brain, cortex, and hippocampus of the mice were measured separately, with the results shown in FIG. 10. The left half brain was further perfused and fixed with pre-cooled 4% paraformaldehyde; and after perfusion, the half brain was taken out and photographed using a camera. Finally, the cortical surface was outlined along the cortical surface frame using ImageJ software and the superficial area of the left half brain of the mice was calculated, with the results shown in FIG. 11.

The results in FIG. 10 show that compared with the control mice, the cKO mice have a significant decrease in the weights of cKO cortex, hippocampus, and half brain, while the weights of the cKO cortex, hippocampus, and half brain of the cKO mice subjected to TPK overexpression treatment are significantly improved.

The results in FIG. 11 show that compared with the control mice, the cKO mice have a significant decrease in the superficial area of the left half brain, while the superficial area of the left half brain of the cKO mice subjected to TPK overexpression treatment is significantly improved.

Conclusion: Injection of the AAV-TPK virus can significantly reduce the neuronal loss in the cKO mice and significantly increase the weights of the cKO cortex, hippocampus, and half brain of the cKO mice, thereby alleviating the brain atrophy of the cKO mice.

### Example 5: In vivo distribution of AAV-CAP-B10 after tail intravenous injection in mice and rescue of TPK protein expression in neuronal cells of cKO mice by AAV-TPK

### I. Test materials

The specific source information of primary and secondary antibodies used in this examples and the following examples is listed in Table 8.

**Table 8**

| Antibody | Source | Molecular weight | Concentration | Item No. | Manufacturer |
|---|---|---|---|---|---|
| Anti-TPK | Rabbit mAb | 27kDa | 1:1000 | ab170863 | Abcam |
| Anti-APP antibody | Rabbit mAb | 100-140kDa | 1:1000 | #2452 | Cell Signaling Technology |
| Anti-BACE1 antibody | Rabbit mAb | 70kDa | 1:1000 | #5606S | Cell Signaling Technology |
| Anti-Tau antibody | Mouse mAb | 50-80kDa | 1:1000 | #4019S | Cell Signaling Technology |
| Anti-pTau-S396 antibody | Rabbit mA | 50-79kDa | 1:1000 | ab32057 | Abcam |
| Anti-β-actin antibody | Rabbit mA | 42kDa | 1:1000 | ab8227 | Abcam |
| Anti-GFP antibody | / | / | 1:500 | ab92456 | Abcam |
| Anti-TPK1 antibody | / | / | 1:100 | ab170863 | Abcam |
| Anti-NeuN antibody | / | / | 1:500 | ab104224 | Abcam |
| Anti-GFAP antibody | / | / | 1:1000 | ab7260 | Abcam |
| Anti-Iba1 antibody | / | / | 1:500 | MA5-27726 | ThermoFisher |
| Anti-4G8 antibody | / | / | 1:500 | MA5-23300 | ThermoFisher |
| Secondary antibody | Goat anti-rabbit IgG (HRP) | / | 1:5000 | cat SA00001 -2 | proteintech |
| Secondary antibody | Goat anti-mouse IgG (HRP) | / | 1:5000 | cat SA00001 -1 | proteintech |
| Secondary antibody | Goat anti-chicken IgY H&L Alexa Fluor 488 | / | 1:500 | ab150169 | Abcam |
| Secondary antibody | Donkey anti-rabbit IgG H&L Alexa Fluor 647 | / | 1:500 | ab15007 5 | Abcam |
| Secondary antibody | Goat anti-mouse IgG H&L Alexa Fluor 555 | / | 1:500 | ab15011 4 | Abcam |

### II. Test methods

### 1. Western blotting detection

1x protease and a phosphatase inhibitor were added to the tissue lysis buffer, and the tissue lysis buffer was precooled on ice. After the weight of the cortex of the left half brains of the mice in the Example 4 was measured, the precooled tissue lysis buffer was added by ratio (about 100 µl added per 10 mg of tissue), 2-3 grinding steel beads were added to the tube, the ep tube was placed in a grinding machine at 4°C and shaken for 100 s. After shaking, the tissue lysis buffer was transferred on ice for lysis for 30 min. After compete lysis, the homogenate was centrifuged using a high-speed centrifuge at 12,000 rpm for 30 min. Then the supernatant was transferred into a newly precooled micro-centrifuge tube and placed on ice to obtain a protein sample, and the precipitate was removed. The protein quantification was carried out using a BCA method, in which, bovine serum albumin (BSA) served as a standard curve. Based on the principle of diurea, Cu²⁺ was reduced to Cu¹⁺ by protein under alkaline conditions, BCA (Bicinchoninic acid) chelated Cu¹⁺ as a color developing agent, and bluish violet was produced, and an absorption peak occurred at 562 nm; and the monovalent Cu¹⁺ was dose-correlated with the protein concentration. The BCA method includes the following specific steps.

BCA agent A and agent B were mixed thoroughly in a ratio of 50:1 to prepare a working solution; 20 µl of the standard samples at concentrations of 0, 0.25 µg/ml, 0.5 µg/ml, 1 µg/ml, and 2 µg/ml were added to a 96-well plate. The samples to be detected were diluted to reach appropriate concentrations, and the total volume of the sample diluent was 20 µL. 200 µL of BCA working solution was added to each well. An ELISA plate was shaken in a shaker and then stood at 37°C for 30 min, and then the absorbance was measured at the wavelength of 562 nm. A standard curve was drawn with the protein content (µg) on the x-axis and the absorbance on the y-axis; and the corresponding protein content (µg) could be obtained from the standard curve according to the measured absorbance of the sample, and the protein content was divided by the total volume of the sample diluent (20 µL) and multiplied by the sample dilution factor to obtain the actual sample concentration (unit: µg/µL).

Then, according to the sample concentration measured by the BCA method, the concentrations of all samples were regulated to 1 µg/µL using the lysis buffer, and loading buffer was added. After thorough mixing, the obtained mixture was placed in a water bath at 95°C and heated for 10 min to prepare the protein sample. Precast gel was inserted into an electrophoresis tank, an electrophoresis buffer was charged, and then a comb was removed; after applying 10 µl of marker from both sides, 10 µl of protein sample was added to each electrophoresis lanes. The voltage was regulated to be only 80 v for migration with stacking glue for 30 min, and the protein separation voltage was regulated to be 120 v for migration with the stacking glue for 80 min. After migration with the glue, membrane transfer was carried out, a sample holder of a membrane transfer tank was mounted according to a sequence of a blackboard (bottom) → a sponge pad → filter paper → gel → a PVDF membrane (pre-soaked in methanol for activation, 30 s) → filter paper → a sponge pad → a white board → an upper clamp (each layer was pressed firmly with a roller); the electrophoretic transfer tank was mounted (placed in ice, ice packs were placed in the tank, and the holder was aligned with the membrane transfer tank black to black, red to red); then the electrophoretic transfer buffer was added, with the level exceeding the PVDF membrane;, and migration was performed at a constant current of 0.2 A for 60 min.

After membrane transfer, the member was blocked in 5% non-fat milk prepared with TBST at room temperature for 2 h. After being blocked, the membrane was cut according to the size of bands, and the anti-TPK antibody, anti-APP antibody, anti-BACE1 antibody, anti-Tau antibody, anti-pTau-S396 antibody, and anti-β-actin antibody were added and cultured overnight in a shaking table at 4°C.

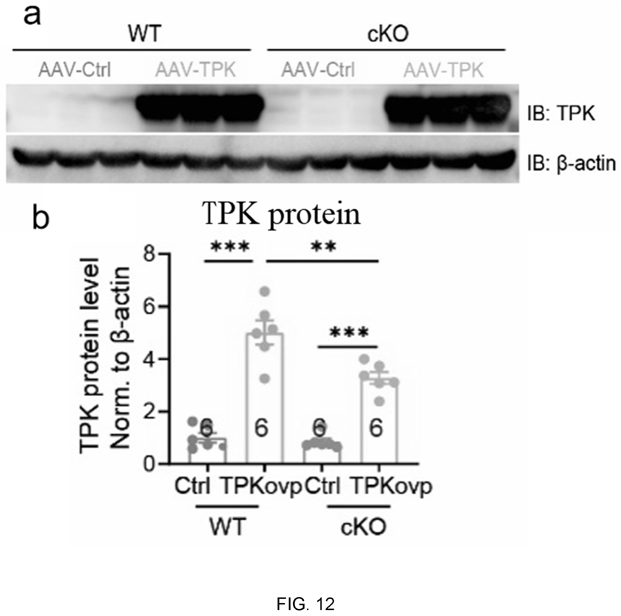

The primary antibodies were extracted on the next day, and the membrane was washed three times with TBST, 10 min each time; then the corresponding second antibodies (goat anti-rabbit IgG and goat anti-mouse IgG) were prepared with 5% non-fat milk powder and cultured for 2 h in the shaking table at room temperature, then the second antibodies were extracted, and the membrane was washed three times with TBST, 10 min each time; after air-drying slightly, it was cultured in an ECL developing solution (solution A: solution B = 1:1) in dark and then was developed using a color developing instrument; the relative content of protein in each sample protein was compared, with the results shown in FIG. 12, in which, FIG. 12a and FIG. 12b respectively show representative images and quantitative results of TPK protein in a cortex sample subjected to western blotting detection 4 weeks after virus infection.

The results in FIG. 12 shows that tail intravenous injection of AAV-TPK can significantly increase the TPK levels in the brain cortexes of both WT mice and cKO mice.

### 2. Immunofluorescence staining detection

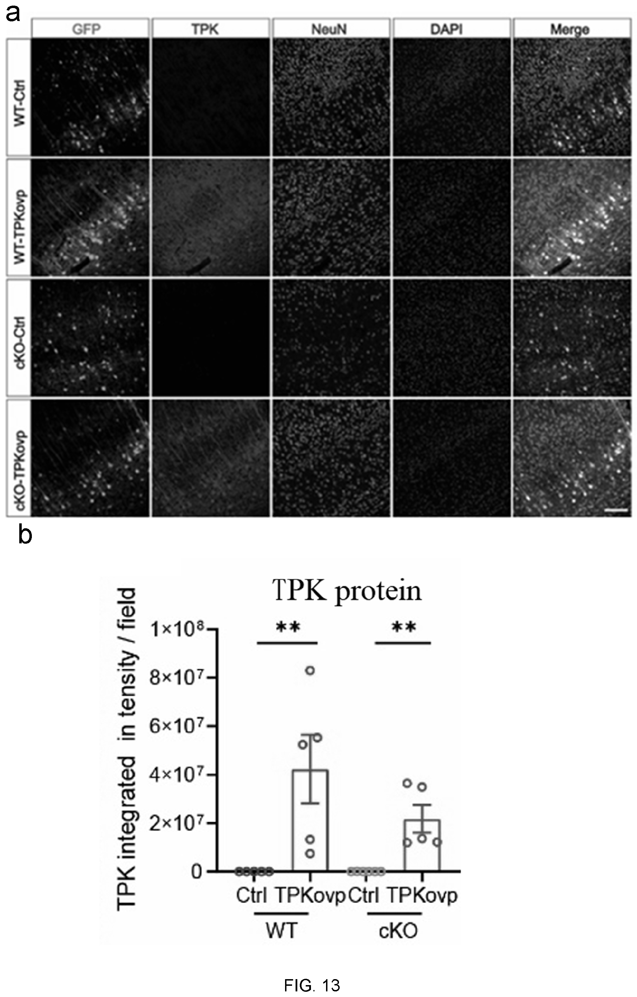

The right half brain of the mice was fixed with 4% paraformaldehyde (PFA) and then subjected to immunofluorescence staining as follows. After being fixed with 4% PFA for 12-24 h, the right half brain was dehydrated and infiltrated in 30% sucrose for 2 days; after being infiltrated in the sucrose, the right half brain was embedded in an optimal cutting temperature compound (OCT) for frozen sections and stored at -80°C. The embedded right half brain was sliced into sections being 30 µm in thickness using a cryostat along the longitudinal axis (coronal angle) from the olfactory bulb to the cerebellum. The sections were washed three times with 1X PBS on the shaker, 10 min each time, to remove the OCT; then 0.5% Triton was added for permeabilization at room temperature for 30 min, followed by blocking with 3% BSA (bovine serum albumin) at room temperature for h; then, the anti-GFP antibody, anti-TPK1 antibody, and anti-NeuN antibody were added for co-staining and cultured overnight at 4°C; the primary antibodies were recovered, and the sections were washed three times with the 1X PBS, 5 min each time; then the corresponding secondary antibodies (goat anti-chicken IgY H&L Alexa Fluor 488, donkey anti-rabbit IgG H&L Alexa Fluor 647, and goat anti-mouse IgG H&L Alexa Fluor 555) were added and cultured at room temperature for 2 h in the dark; finally, the sections were applied on slides; and after air-drying slightly, the sections were covered with a dropwise-added mounting medium and imaged using a Nikon Ti2E inverted microscope. The results are shown in FIG. 13, in which, FIG. 13a and FIG. 13b respectively show representative images and quantitative results of the TPK protein in cortical samples subjected to immunofluorescence staining detection 4 weeks after AAV delivery.

The results in FIG. 13 show that GFP is expressed in all groups, but TPK is strongly expressed only in the TPKovp groups (including WT-TPKovp and cKOTPKovp groups), which is consistent with changes in protein levels.

Conclusion: Tail intravenous injection of AAV-TPK can significantly increase TPK levels in the cortex of the cKO mice.

### Example 6: In vivo distribution of AAV-CAP-B10 in mice after tail intravenous injection

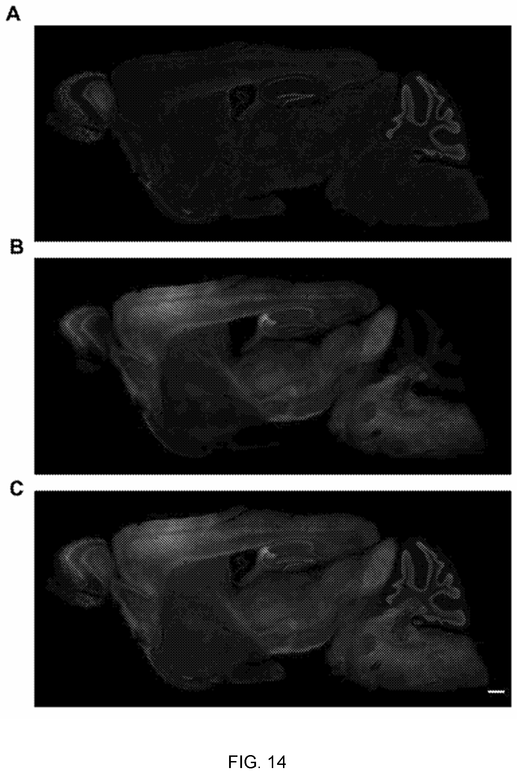

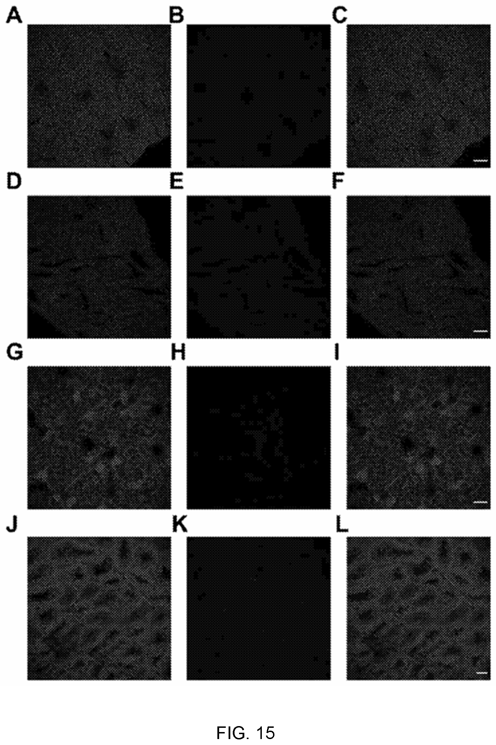

The purpose of this example is to investigate the in vivo distribution of AAV.CAP-B10 virus in various organs of mice after peripheral administration. The method the same as in the above example was utilized; the mice were administered with AAV-CAP-B10 by tail intravenous injection, then the tissue sections of each organ of the mice were subjected to fluorescence protein signal detection, with the results shown in FIG. 14 to FIG. 15. In which, FIG. 14 is a representative confocal image of mouse brain sections after brain infection with an AAV.CAP-B10 virus; and FIG. 15 is representative confocal images of mouse liver, heart, kidney, and testis tissue sections after infection with an AAV.CAP-B10 virus.

In FIG. 14, FIG. 14A shows DAPI signal; FIG. 14B shows virus-expressed fluorescent protein signal; FIG. 14C combines FIG. 14A and FIG. 14B and shows the areas of viral expression. The scale bar is 500 µm.

In FIG. 15, FIG. 15A-C are liver tissue images, in which, FIG. A shows DAPI signal, FIG. B shows: virus-expressed fluorescence protein signal, FIG. C combines FIG. A and FIG. B and shows the areas of viral expression; FIG. 15D-F are heart tissue images; FIG. 15G-I are kidney tissue images; FIG. 15J-L are testis tissue images. The order of each row is the same as the first row. The scale bar is 100 µm.

The results in FIG. 14 to FIG. 15 show that after tail intravenous injection of AAV-CAP-B10, the virus is expressed in the mouse brain tissue, but not expressed in the liver, heart, kidney, and testis tissues of the mice.

Conclusion: Peripheral administration of the AAV.CAP-B10 virus can specifically target in brain without expression in peripheral organs. This operation is convenient and can avoid the potential side effects of peripheral TPK overexpression.

### Example 7. Tail intravenous injection of AAV-TPK on alleviation of multiple pathological changes in cKO mice

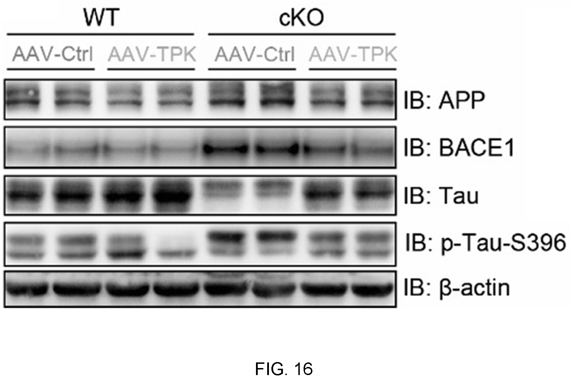

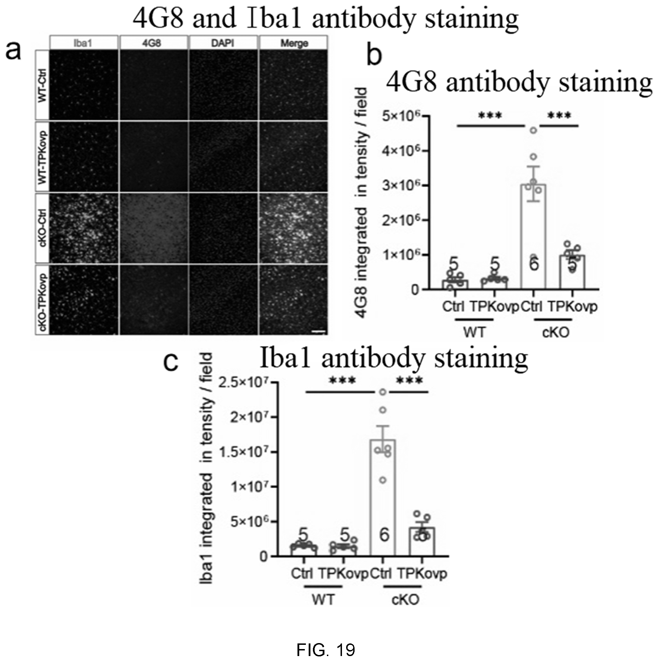

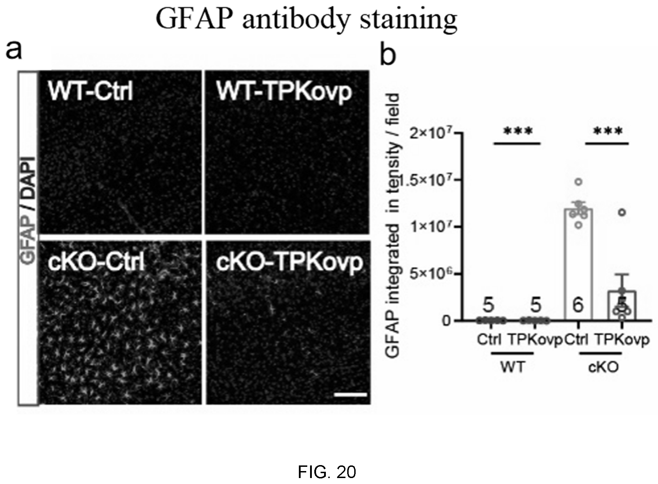

In order to investigate the neuronal loss and brain inflammation activation, the brain sections in the Example 5 were cultured with the anti-NeuN antibody and anti-GFAP antibody by the same method; on the other hand, the anti-Iba1 antibody and anti-4G8 antibody were utilized to co-stain. The protein blotting detection and immunofluorescence staining detection were carried out by the methods the same as in the Example 5, with the results shown in FIG. 16 to FIG. 20.

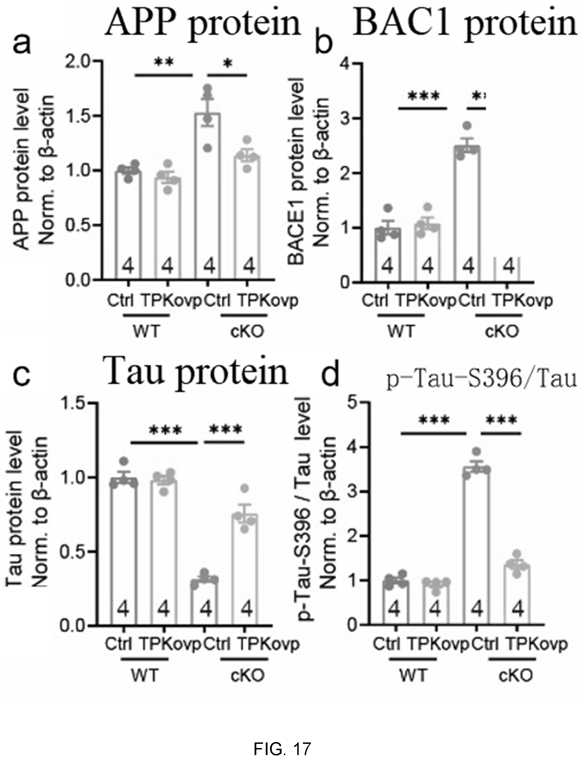

FIG. 16 and FIG. 17 collectively show the representative images and quantitative results of APP, BACE1, Tau, and p-Tau(S396) subjected to Western blotting detection.

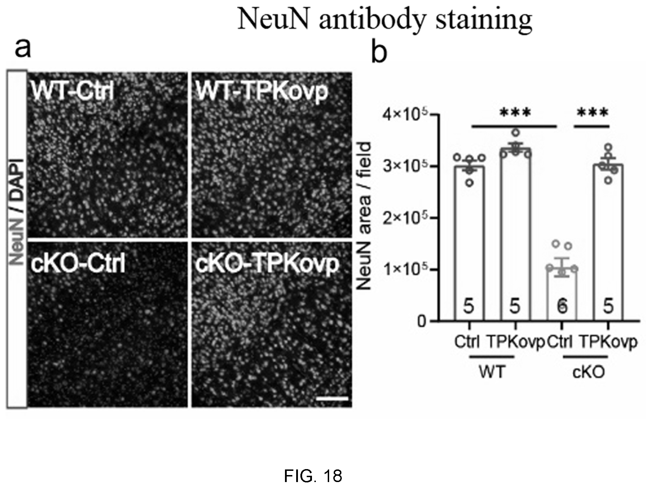

FIG. 18 to FIG. 20 collectively show the representative images and quantitative results of immunofluorescence staining with NeuN, 4G8, Iba1, and GFAP antibody.

The results in FIG. 16 to FIG. 17 show that compared with the littermate control mice, the cKO mice have a significant increase in APP and BACE1 protein levels, p-Tau-S396, and p-Tau/Tau ratio, while the total Tau protein levels decrease; and these changes can be significantly alleviated by TPK overexpression treatment
The results in FIG. 18 to FIG. 20 show that compared with the littermate control mice, the cKO mice have significant neuronal loss, enhanced Aβ, and activation of microglia and astrocytes; and these changes can be significantly alleviated by TPK overexpression treatment.

Conclusion: Tail intravenous injection of AAV-TPK can effectively alleviate the elevation of APP and BAC1, the increase of p-Tau-S396, and the decrease of total Tau caused by reduced TPK levels, thereby effectively reversing these pathological changes; in addition, it can also effectively reverse the neuronal loss of the cKO mice, alleviate the activation of astrocytes and microglia caused by reduced TPK levels, and effectively reduce the deposition of Aβ plaques.

The above are only preferred examples of the present invention and do not constitute any limitation on the form or substance of the present invention. It is to be noted that, for those skilled in the art, several improvements and supplements can be made without departing from the premise of the present invention, and these improvements and supplements should also be regarded as falling within the scope of protection of the present invention. Any slight modifications, embellishments, and equivalent variations made by technical personnel familiar with the profession using the technical content disclosed above, without departing from the spirit and scope of the present invention, are considered equivalent embodiments of the present invention; meanwhile, any equivalent modifications, embellishments, and evolutions made to the above embodiments based on the substantive technology of the present invention still fall within the scope of the technical solutions of the present invention.

## Claims

1. Intravenous injection of an adeno-associated virus with overexpression of thiamine pyrophosphokinase-1, the adeno-associated virus with overexpression of thiamine pyrophosphokinase-1 being a recombinant AAV, wherein a genome of the adeno-associated virus with overexpression of thiamine pyrophosphokinase-1 comprises a neuron-specific promoter, a nucleotide sequence encoding a TPK gene, and a WPRE element.

2. The intravenous injection of the adeno-associated virus with overexpression of thiamine pyrophosphokinase-1 according to claim 1, wherein the serotype of AAV is AAV.CAP-B10, the neuron-specific promoter has a gene sequence shown as SEQ ID NO: 2, the nucleotide sequence encoding the TPK gene is shown as SEQ ID NO: 3, and the WPRE element has a sequence shown as SEQ ID NO: 4.

3. The intravenous injection of the adeno-associated virus with overexpression of thiamine pyrophosphokinase-1 according to claim 1, wherein the sequence of the recombinant AAV is shown as SEQ ID NO: 1.

4. A method for constructing the adeno-associated virus with overexpression of thiamine pyrophosphokinase-1 according to claim 1, comprising the following steps:
step 1: obtaining a TPK target gene by a PCR amplification or restriction endonuclease digestion method, and performing homologous recombination using Exnase recombinase, then assembling with an adeno-associated virus vector by a T4 DNA linkage method;
step 2: transforming the virus vector assembled in step 1 into E.coli Stbl3 competent cells;
step 3: screening a transformant by colony PCR, sequencing positive clones, and determining the positive clones by sequence alignment; and
step 4: the screened and identified virus vector together with pHelper plasmid and pAAV-R plasmid co-transfecting with AAV-293 cells, and then performing packaging and purifying of the AAV.

5. The method for constructing the adeno-associated virus with overexpression of thiamine pyrophosphokinase-1 according to claim 4, wherein primers for the PCR amplification in step 1 or colony PCR in step 3 are a 4423-BamHI-F primer and a 4423-SalI-R primer, the sequence of the 4423-BamHI-F primer is shown as SEQ ID NO: 5, and the sequence of the 4423-SalI-R primer is shown as SEQ ID NO: 6.

6. The method for constructing the adeno-associated virus with overexpression of thiamine pyrophosphokinase-1 according to claim 4, wherein a forward sequencing primer and a reverse sequencing primer for transformant sequencing in step 3 are a hSyn-C-F primer and a pEGFP-N-3' primer, respectively, the sequence of the hSyn-C-F primer is shown as SEQ ID NO: 7, and the sequence of the pEGFP-N-3' primer is shown as SEQ ID NO: 8.

7. A kit for preparing the adeno-associated virus with overexpression of thiamine pyrophosphokinase-1 according to claim 1, comprising a 4423-BamHI-F primer and a 4423-SalI-R primer for PCR amplification, wherein the sequence of the 4423-BamHI-F primer is shown as SEQ ID NO: 5, and the sequence of the 4423-SalI-R primer is shown as SEQ ID NO: 6.

8. The kit for preparing the adeno-associated virus with overexpression of thiamine pyrophosphokinase-1 according to claim 7, further comprising the forward sequencing primer and the reverse sequencing primer for detecting the positive clones, wherein the forward sequencing primer is the hSyn-C-F primer and has the sequence shown as SEQ ID NO: 7, and the reverse sequencing primer is the pEGFP-N-3' primer and has the sequence shown as SEQ ID NO: 8.

9. An application of the adeno-associated virus with overexpression of thiamine pyrophosphokinase-1 according to claim 1 in preparation of a medicament for preventing or treating Alzheimer's disease.

10. An application of the adeno-associated virus with overexpression of thiamine pyrophosphokinase-1 according to claim 9 in preparation of a medicament for preventing or treating Alzheimer's disease-like neurodegeneration, brain atrophy, Aβ and tau pathological changes, neuroinflammation, neurovascular disorders, or slowing down progression of Alzheimer's disease.
